# EUROPÄISCHE PATENTSCHRIFT

(11) **EP 4 107 747 B1**
(45) Veröffentlichungstag und Bekanntmachung des Hinweises auf die Patenterteilung: **03.09.2025**
(21) Anmeldenummer: 21706926.9
(22) Anmeldetag: 19.02.2021
(51) Int. Cl.: G16H 40/63

(54) **SICHERHEITSRELEVANTE ANWENDUNG**
SAFETY-RELEVANT APPLICATION
APPLICATION RELATIVE À LA SÉCURITÉ

(30) Priorität: 21.02.2020 DE 102020104665
(43) Veröffentlichungstag der Anmeldung: 28.12.2022
(73) Patentinhaber: B. Braun Melsungen AG, 34212 Melsungen (DE)
(72) Erfinder: ERLEN, Christoph, 34123 Kassel (DE); GREEN, James Richard, Chester Cheshire 4 8BG (GB); HÖLLERICH, Mike, 50968 Köln (DE)
(74) Vertreter: Winter, Brandl - Partnerschaft mbB
(86) Internationale Anmeldenummer: PCT/EP2021/054114
(87) Internationale Veröffentlichungsnummer: WO 2021/165449

(56) Entgegenhaltungen:
- WO-A1-2016/205051

## Beschreibung

Die Erfindung betrifft sicherheitsrelevante, vorzugsweise medizinische Anwendungen, insbesondere eine Vorrichtung für eine sicherheitsrelevante, vorzugsweise medizinische Anwendung und ein Verfahren zur Bedienung einer solchen.

### Stand der Technik

Aus dem Stand der Technik sind bereits eine Vielzahl von Medizinprodukten bekannt, die eine Eingabe numerischer Werte durch Benutzer, wie Krankenschwestern und Ärzte, an Benutzeroberflächen als Teil einer Benutzerschnittstelle einer medizinischen Vorrichtung erfordern. In vielen Fällen sind die eingegebenen Daten oder Parameter sicherheitskritisch, hierin auch als sicherheitsrelevant bezeichnet, was bedeutet, dass eine falsche Eingabe zu Gefahren für den Patienten, den Bediener oder andere führen kann oder führt.

Bei sicherheitskritischen Vorrichtungen, wie einer Infusionspumpe, ist die Durchflussmenge ein Beispiel eines sicherheitskritischen Parameters. Eine falsche Eingabe der Durchflussmenge kann zu Dosierungsfehlern führen. Abhängig vom infundierten Medikament kann dies dem Patienten schweren Schaden zufügen.

Der Stand der Technik hat jedoch immer den Nachteil, dass die Benutzerfreundlichkeit und nicht die maximale Sicherheit im Vordergrund steht.

Es besteht Bedarf zur Bereitstellung von Konzepten zur Erhöhung des Sicherheitsniveaus bei sicherheitsrelevanten Anwendungen, insbesondere bei solchen, bei denen numerische Werte durch den Bediener eingebbar sind.

WO 2016/205051 A1 offenbart eine Maschine zur Behandlung medizinischer Flüssigkeiten, umfassend: eine Pumpe zum Abpumpen der medizinischer Flüssigkeit; ein Eingabegerät, das dazu konfiguriert ist: Anzeigen von Berührungsschaltflächen, wobei zumindest einige der Berührungsschaltflächen jeweils ein Graphem darstellen, und Erfassen haptischer Interaktionen zwischen einem Benutzer und den Berührungsschaltflächen, wobei eine Interaktion bewirkt, dass Informationen bezüglich der Berührungsschaltfläche von der medizinischen Fluidbehandlungsmaschine empfangen werden; und eine Steuereinheit, die dazu konfiguriert ist, das Eingabegerät zu steuern, wobei das Steuern des Eingabegeräts beinhaltet, dass das Eingabegerät veranlasst wird, zwischen dem Anzeigen i) eines ersten Satzes von Berührungsschaltflächen, die einer ersten Sprache entsprechen, und ii) eines zweiten Satzes von Berührungsschaltflächen umzuschalten, die einer ersten Sprache entsprechen einer Zweitsprache entsprechen.

### Kurzbeschreibung der Erfindung

Es ist also die Aufgabe der Erfindung, die Nachteile aus dem Stand der Technik zu vermeiden oder wenigstens zu mildern. Insbesondere soll das Sicherheitsniveau bei der Eingabe numerischer Werte an sicherheitsrelevanten Anwendungen erhöht/verbessert werden.

Diese Aufgabe wird dadurch gelöst, dass eine Vorrichtung für eine sicherheitsrelevante Anwendung bereitgestellt wird. Die Vorrichtung hat eine Benutzerschnittstelle. Die Benutzerschnittstelle ist vorgesehen, eine vorbestimmte Anzahl an Digits anzuzeigen. Die vorbestimmte Anzahl an Digits ist vorgesehen, von einem Benutzer mit numerischen Werten belegt zu werden. Die Benutzerschnittstelle ist ausgebildet, eine Auswahl vorbestimmten Anzahl an Digits, die für eine Benutzereingabe zur Verfügung stehen, zu beschränken, bei einer Eingabe eines numerischen Wertes durch den Benutzer, zum Beispiel seine/ihre erste Eingabe, an einem der zu belegenden/zur Verfügung stehenden Digits.

Durch das Einschränken der Möglichkeit, Digits mit Werten zu belegen, wird der Benutzer proaktiv daran gehindert, falsche Werte einzugeben und somit Schaden zu verursachen.

Insbesondere werden hierin Benutzerschnittstellen zur Eingabe von Daten durch einen Benutzer einer Maschine verstanden. Die Benutzerschnittstelle kann außerdem die Stelle sein, mit der ein Mensch mit einer Maschine oder einem Gerät, hierin die Vorrichtung für die sicherheitsrelevante Anwendung, in Interaktion tritt.

Die sicherheitsrelevante Anwendung kann beispielsweise das Verabreichen/Infundieren von Medikamenten oder dergleichen sein. Allgemein kann die sicherheitsrelevante Anwendung eine Anwendung verstanden werden, bei der der Mensch bei Falscheingabe oder Falschbedienung der Benutzerschnittstelle direkter Gefahr oder Schaden ausgesetzt ist.

Die Sicherheitsaspekte der Vorrichtung für die sicherheitsrelevante Anwendung können in drei Kategorien eingeteilt sein. Primäre Sicherheit umfasst Risiken, die unmittelbar von einem System ausgehen, zum Beispiel ein durch einen Fehler in der Elektronik verursachter Brand. Funktionelle Sicherheit umfasst Gefährdungen, die von der Vorrichtung selbst ausgehen können, zum Beispiel abhängig von einer korrekten Funktion von Hardware oder Software der Vorrichtung. Indirekte Sicherheit umfasst sowohl indirekte Auswirkung von Fehlern der Vorrichtung als auch Mängel im Mensch-Maschine Interface, hier die Benutzerschnittstelle. Auch nicht funktionale Eigenschaften wie beispielsweise die Bearbeitungsdauer einer Anforderung gehören zu dieser Kategorie. Somit kann die Sicherheitsrelevanz vorliegend vor allem die indirekte Sicherheit als nicht-funktionale Eigenschaft der Vorrichtung betreffen.

Zum Verständnis, Digits werden als in der Anzeige des elektronischen Geräts, nämlich die Benutzerschnittstelle, vorhandene Stellen zur Belegung mit Werten verstanden. Hierin kann die Eingabe auf numerische Werte, also Zahlen von 0 bis 9, beschränkt sein.

Vorteilhafte Ausführungsformen sind in den Unteransprüchen beansprucht und werden nachfolgend näher erläutert.

Die Vorrichtung kann eine medizinische Vorrichtung sein. Beispiele hierfür sind eine Infusionspumpe oder eine Dialysemaschine. Die sicherheitsrelevante Anwendung steht somit in direktem Bezug zum Menschen und birgt eine Gefahr für Leib und Leben.

Die Benutzerschnittstelle der Vorrichtung kann ein Touchpad zur manuellen Eingabe und Ausgabe sein. Ferner kann die Benutzerschnittstelle eine Maus zur Eingabe aufweisen. Die Maus kann in Verbindung mit dem Touchpad oder mit einem ohne Touchfunktion ausgestatteten Bildschirm verwendet werden. Ebenfalls kann die Benutzerschnittstelle ein Touchscreen zur manuellen Ein-/Ausgabe aufweisen oder sein.

Eine Eingabe von numerischen Werten kann hierüber erleichtert werden.

Die zu belegenden Digits können separat mit numerischen Werten belegbar sein. Ebenfalls können die zu belegenden Digits nacheinander belegbar sein. Diesbezüglich sei angemerkt, dass die Digits so ausgelegt sein können, dass sie ausschließlich einzeln durch den Benutzer mit einem jeweiligen numerischen Wert belegbar sind.

Hierdurch kann die Gefahr einer Falscheingabe weiter reduziert werden.

Die Benutzerschnittstelle kann ferner ausgebildet sein, die Auswahl für die Benutzereingabe zur Verfügung stehender Digits auf eine gleiche Anzahl an Digits für Vorkommastellen und Nachkommastellen zu verteilen. Die Benutzerschnittstelle kann ebenfalls oder zusätzlich ausgebildet sein, die Auswahl für die Benutzereingabe zur Verfügung stehender Digits auf eine gleiche Anzahl an Digits für eine Einheit und eine Untereinheit, vorzugsweise Stunden und Minuten bzw. Minuten und Sekunde, zu verteilen. Somit können zum Beispiel lediglich vier Digits mit Werten belegt werden. Bei Eingabe einer Einheit kann das Hinzufügen einer weiteren Untereinheit von einer Untereinheit gesperrt sein. Dasselbe kann gelten bei der Eingabe einer Untereinheit und einer Untereinheit dazu, dass die Obereinheit gesperrt ist. Diesbezüglich können zum Bespiel auch zwei Vorkommastellen und zwei Nachkommastellen belegbar sein, aber keine ungerade Aufteilung.

Die Benutzerschnittstelle kann ferner ausgebildet sein, mittels Eingabe durch den Benutzer einen auf der Benutzerschnittstelle zur Anzeige von Vorkommastellen und Nachkommastellen vorgesehenen Dezimalpunkt zwischen den zu belegenden Digits zu verschieben. Hierzu können die Nachkommastellen-Digits kleiner als die Vorkommastellen-Digits angezeigt sein. Durch Verschieben des Dezimalpunktes kann der Benutzer diesen auch verschwinden lassen, so dass der Benutzer durch Verschieben des Dezimalpunktes auf der Anzeige der Benutzerschnittstelle lediglich Vorkommasteilen-Digits auswählt.

Ebenfalls kann das/der Touchpad/Touchscreen ausgebildet sein, bei Anklicken eines jeweiligen zu belegenden Digits durch den Benutzer, eine Tastatur anzuzeigen, die vorgesehen ist, bei Berührung eines entsprechend auf der Tastatur angezeigten numerischen Werts, das jeweils zu belegende Digit mit diesem Wert zu belegen.

Die Vorrichtung kann ferner einen Speicher aufweisen. Der Speicher kann vorgesehen sein, Daten zu speichern. Die Daten können mindestens einen harten numerischen Grenzwert und einen weichen numerischen Grenzwert umfassen. Diese Grenzwerte können sich auf die sicherheitsrelevante Anwendung beziehen. Ebenfalls können hierfür jeweilige harte und weiche Ober- und Untergrenzen in Bezug auf die sicherheitsrelevante Anwendung in dem Speicher gespeichert sein. Die Benutzerschnittstelle kann in Kommunikation mit dem Speicher stehen. Diese Kommunikation kann direkt oder indirekt über ein in der Vorrichtung enthaltene Verarbeitungseinheit oder Steuereinheit hergestellt sein. Die Verarbeitungseinheit bzw. Steuereinheit kann im Sinne eines Netzwerks, zum Beispiel Bus, ausgebildet sein, Daten von dem Speicher und der Benutzerschnittstelle zu verarbeiten. Die Benutzerschnittstelle kann ausgebildet sein, basierend auf den harten und weichen numerischen Grenzwerten, einen Eingabefortschritt zu verzögern oder zu verhindern. Dies kann auf einem durch die Verarbeitungseinheit oder Steuereinheit bereitgestellten Befehl an die Benutzerschnittstelle basieren.

Die Benutzerschnittstelle kann ausgebildet sein, basierend auf einem Vergleich zwischen mindestens einem der mit numerischen Werten belegten Digits und dem mindestens einen harten numerischen Grenzwert eine weitere Eingabe zu beschränken oder eine vorherige Eingabe rückgängig zu machen. Ebenfalls kann die Benutzerschnittstelle ausgebildet sein, basierend auf dem Vergleich zwischen dem mindestens einen der mit numerischen Werten belegten Digits und dem mindestens einen harten numerischen Grenzwert die Auswahl für die Benutzereingabe zur Verfügung stehender Digits und/oder einen zur Auswahl stehenden numerischen Wertebereich an dem mindestens einen der zur Verfügung stehenden Digits zu beschränken. Die Verarbeitung im Sinne des Vergleichs kann durch die Verarbeitungseinheit oder Steuereinheit durchgeführt werden.

Hierdurch lässt sich, durch geeignete Beschränkungen und/oder Verzögerungen, die Aufmerksamkeit des Benutzers auf das Gerät richten.

Die Benutzerschnittstelle kann zusätzlich ausgebildet sein, basierend auf einem Vergleich zwischen dem mindestens einen der mit numerischen Werten belegten Digits und dem mindestens einen weichen Grenzwert, mitzuteilen, dass eine weitere Eingabe erforderlich ist. Hierbei kann der Wert in dem mindestens einen der mit numerischen Werten belegten Digits oder ein Gesamtwert aller belegter Digits innerhalb der mindestens einen harten Grenze oder innerhalb eines durch die harte Obergrenze und Untergrenze definierten Bereichs liegen, aber außerhalb der mindestens einen weichen Grenze oder außerhalb eines durch die weiche Obergrenze und Untergrenze definierten Bereichs.

Somit kann eine Patientensicherheit weiter erhöht werden.

Die oben genannte Aufgabe kann ferner durch ein Verfahren zum Bedienen einer Vorrichtung für eine sicherheitsrelevante Anwendung gelöst werden. Die Vorrichtung kann eine wie oben beschriebene Vorrichtung sein. Das Verfahren umfasst Anzeigen, durch eine Benutzerschnittstelle, von einem Benutzer mit numerischen Werten zu belegender Digits. Das Verfahren umfasst ferner Eingeben eines numerischen Werts, durch einen Benutzer, an einem der zu belegenden/zur Verfügung stehender Digits. Das Verfahren umfasst außerdem Beschränken, durch die Benutzerschnittstelle, einer Auswahl für eine Benutzereingabe zur Verfügung stehender Digits basierend auf dem Eingeben. Hierdurch kann durch das Eingeben eines Wertes an einem der angezeigten Digits an der Benutzerschnittstelle eine weitere Eingabe eingeschränkt und/oder verzögert werden.

Ein Sicherheitsaspekt beim Bedienen sicherheitsrelevanter Vorrichtungen kann somit verbessert werden.

Mit anderen Worten betrifft die Erfindung einen numerischen Editor für sicherheitskritische Anwendungen auf Touchscreen-Benutzeroberflächen, zum Beispiel in Form einer Benutzerschnittstelle. Insbesondere betrifft die Erfindung ein Editor-Konzept für Touchscreen-Benutzeroberflächen, das die Wahrscheinlichkeit falscher Eingaben auf ein Minimum reduzieren soll. Das in Konsumgütern wie Smartphones verwendete Eingabekonzept dient in erster Linie der Benutzerfreundlichkeit und nicht der maximalen Sicherheit. Das vorgeschlagene Konzept bietet ein ausgewogenes Verhältnis zwischen Sicherheit und Ergonomie. Es kann unter Berücksichtigung einer medizinischen Infusionspumpe verwendet werden, die sicherheitskritisch ist und eine begrenzte Bildschirmgröße aufweist. Das Konzept ist jedoch nicht auf Infusionspumpen beschränkt. Eine Anwendung in anderen sicherheitskritischen Geräten im Gesundheitswesen und darüber hinaus ist denkbar.

Mit noch anderen Worten kann die Erfindung eine technische Gerätschaft sein, die eine Steuereinheit, eine damit verbundene Bedieneinheit und eine mit der Steuereinheit ebenfalls verbundene Ausgabeeinheit aufweist. Die Bedieneinheit kann die Eingabe von numerischen Parametern durch einen Benutzer erfordern und die Steuereinheit diese Eingabe zum Erzeugen eines Sollwerts für eine Ausgabeeinheit verwenden.

In einer oder mehreren Ausführungsformen kann eine spezifische Eingabevorrichtung, auch Editor genannt, in der Bedieneinheit vorgesehen sein, die die Wahrscheinlichkeit der Fehleingaben der numerischen Parameter minimieren soll.

Es können verschiedene Varianten für die technische Gerätschaft vorgesehen sein. Beispielsweise kann die technische Gerätschaft eine Vorrichtung sein, deren Zweck die Durchführung, Steuerung und/oder Dokumentation eines oder mehrerer sicherheitskritischer Vorgänge ist, der/die bei Eingabefehlern zu Gefährdungen von Menschen, Gütern, der Umwelt und/oder finanziellen Werten führen kann/können.

Insbesondere kann der Zweck der Vorrichtung die Verabreichung und/oder Dokumentation von medizinischen Infusionen. Außerdem kann der Zweck der Vorrichtung die Durchführung und/oder Dokumentation von Dialysebehandlungen, einer Therapie mittels Temperatur, einer Therapie mittels Ultraschall, einer Therapie mittels elektromagnetischer Strahlen (inklusive Infrarot und Röntgen), von Partikeltherapie und/oder von Röntgendiagnostik sein.

Ebenso kann der Zweck der Vorrichtung die Steuerung eines sicherheitskritischen Vorgangs eines Fahrzeugs, eines Luftfahrzeugs, eines Waffensystems, von Industrieanlagen, von Geschäftsabläufen, von Verkehrsflüssen, eines Kraftwerks sein.

Die Steuereinheit kann eine Vorrichtung mit einem programmierbaren Prozessor nebst Speicher sein. Diese kann zusätzlich mit einer Ablauflogik, zum Beispiel zur Durchführung von Infusionen oder Dialysebehandlungen, versehen sein, die auf dem Prozessor ausgeführt wird und die Eingabe von Parametern erfordert.

Die Bedieneinheit, als zumindest ein Teil der Benutzerschnittstelle, kann eine Vorrichtung mit einer visuellen Ausgabefläche und ein auf dieser Fläche befindliches berührempfindliches Eingabemedium sein. Die Ausgabefläche kann Bedienelemente anzeigen, wobei die Detektion von Berührungen zum Auslösen spezifischer Reaktionen in der Bedieneinheit und der Steuereinheit führen kann. Hierzu können resistive, kapazitive, Kraft-basierte und/oder Temperatur-basierte Touchsensoren verwendet werden. Ebenfalls kann eine Kopplung der Ausgabefläche mit der Eingabe mittels einer Computermaus oder einem Trackpad, mittels Gestensteuerung und/oder mittels kamerabasierter Blickerkennung (englisch für eye-tracking) vorgesehen sein. Die Bedieneinheit kann dabei zumindest ein Teil eines Tablets oder Personalcomputers sein.

Die Ausgabeeinheit kann ein Stellglied/Motor aufweisen, das/der Bewegungen gemäß der Vorgabewerte der Steuereinheit ausführt. Das Stellglied kann mit einer Spritze gekoppelt sein, so dass eine Infusion kontrolliert verabreicht werden kann. Der Motor kann mit einem Infusionsschlauch mittels einer Peristaltik gekoppelt sein, so dass eine Infusion kontrolliert verabreicht werden kann. Ferner kann die Ausgabeeinheit einen Generator von Röntgenstrahlung, elektromagnetischen Wellen, Partikelströmen und/oder Dokumentationselementen aufweisen.

Die zur Eingabe vorgesehenen numerischen Parameter in Form numerischer Wertebereiche können sicherheitskritische Zahlenwerte sein, wie Einstellparameter einer Infusionstherapie (z.B. Flussrate, zu verabreichendes Volumen, Dauer der Infusion, Bolusmenge, Medikamentenkonzentration und/oder Dosisraten) oder Dialysebehandlung. Ebenfalls können die sicherheitskritischen Zahlenwerte sicherheitskritische Angaben von Zeit, Energiemengen, Leistungswerten und/oder Geschwindigkeiten sein.

Die Benutzer können Führer von sicherheitskritischen Abläufen sein, die zur Bedienung der technischen Gerätschaft erforderlich sind. Hierunter fallen zum Beispiel medizinisches Personal (inklusive Krankenpfleger und Ärzte), Krankenhaus Techniker, Wartungspersonal, Benutzer einer Maschine, Benutzer eines Fahrzeuges und Benutzer einer Anlage.

Gemäß einer Ausführungsform kann die spezifische Eingabevorrichtung, der Editor, wie folgt ausgeführt sein. Dezimalstellen des numerischen Parameters können so dargestellt werden, dass sie gemeinsam sichtbar, aber einzeln auf der Bedieneinheit auswählbar sind. Zum Beispiel erst bei Anwahl eine Dezimalstelle durch den Benutzer, wird eine Tastatur mit einem Zahlenstrahl angezeigt und aktiviert. Die Tastatur kann die Festlegung des Werts an der ausgewählten Dezimalstelle erlauben. Nach Auswahl des Werts kann die Tastatur auf der Bedieneinheit wieder ausgeblendet und deaktiviert werden. Die Dezimalstellen, die rechts von der editierten Zahlenstelle liegen, können nach der Auswahl des Werts mit Nullen gefüllt werden, wenn sie zuvor nicht bereits mit spezifischen Werten durch den Benutzer belegt worden sind. Zusätzlich kann die Tastatur auch ein Eingabeelement zum Löschen des Werts an der ausgewählten Dezimalstelle aufweisen. Wenn einzelne Werte auf der Tastatur zu ungültigen Eingaben führen, werden beispielsweise die zugehörigen Bedienelemente deaktiviert und deren Deaktivierung dem Anwender angezeigt (z.B. durch Ausgrauen).

Alternativ werden diese Werte gar nicht erst auf der Tastatur angezeigt. Zusätzlich kann ein Bedienelement zum Löschen/Zurücksetzen des kompletten Parameterwerts vorgesehen sein. Ferner können unterschiedliche Anzeigegrößen für Dezimalstellen vor und nach dem Komma vorgesehen sein, um Verwechslung der Zahlenstellen vorzubeugen. Weitere Bedienelemente können zum Verschieben des Dezimalpunkts vorgesehen sein. Ebenfalls können keine Dezimalpunkte vorhanden sein. Auch eine zusätzliche Anzeige von abhängigen numerischen Parametern kann während des Bedienvorgangs. vorgesehen sein. Außerdem kann eine Kennzeichnung, um welchen numerischen Parameter es sich handelt (mittels eines Parameternamens, grafischen Elementen und/oder der Angabe der technischen Einheit) vorgesehen sein.

Ein noch weiteres Bedienelement kann zum Bestätigen des Wertes, nachdem die Eingabe des numerischen Parameters vom Benutzer abgeschlossen wurde, vorgesehen sein. Eine Prüfung auf zulässige Wertbereiche durch die Bedien- und/oder die Steuereinheit kann beim Bestätigen vorgesehen sein. Die Prüfung kann durch die Bedien- und/oder die Steuereinheit auf unter Sicherheitsgesichtspunkten auffällige Wertbereiche (Soft-Limit-Bereich) beim Bestätigen durchgeführt werden. Es kann eine Rückfrage beim Benutzer durchgeführt werden, ob der Wert wirklich anzuwenden ist, wenn der Wert im auffälligen Wertebereich liegt. Es kann ebenso eine akustische Ausgabe des Prüfungsergebnisses erfolgen. Eine grafische Anzeige der zulässigen bzw. auffälligen Wertebereiche auf einem Zahlenstrahl kann auch erfolgen.

Es ist dem Fachmann klar, dass die hierin dargelegten Erklärungen unter Verwendung von Hardwareschaltungen, Softwaremitteln oder einer Kombination davon implementiert sein/werden können. Die Softwaremittel können im Zusammenhang stehen mit programmierten Mikroprozessoren oder einem allgemeinen Computer, einer ASIC (Englisch: Application Specific Integrated Circuit; zu Deutsch: anwendungsspezifische integrierte Schaltung) und/oder DSPs (Englisch: Digital Signal Processors; zu Deutsch: digitale Signalprozessoren).

Beispielsweise [ kann die Vorrichtung für die sicherheitsrelevante Anwendung, die Benutzerschnittstelle, der Speicher, die Verarbeitungseinheit und die Steuereinheit teilweise als ein Computer, eine Logikschaltung, ein FPGA (Field Programmable Gate Array; zu Deutsch: im Feld programmierbare Logik-Gatter-Anordnung), ein Prozessor (beispielsweise umfassend einen Mikroprozessor, einen Mikrocontroller (µC) oder einen Vektorprozessor)/Core (zu Deutsch: Hauptspeicher, kann in dem Prozessor integriert sein beziehungsweise von dem Prozessor verwendet werden)/CPU (Englisch: Central Processing Unit; zu Deutsch: zentrale Prozessoreinheit; wobei mehrere Prozessorkerne möglich sind), eine FPU (Englisch: Floating Point Unit; zu Deutsch: Gleitkommaprozessoreinheit), eine NPU (Englisch: Numeric Processing Unit; zu Deutsch: Numerische Prozessoreinheit), eine ALU (Englisch: Arithmetic Logical Unit; zu Deutsch: arithmetisch-logische Einheit), ein Koprozessor (zusätzlicher Mikroprozessor zur Unterstützung eines Hauptprozessors (CPU)), eine GPGPU (Englisch: General Purpose Computation on Graphics Processing Unit; zu Deutsch: Allzweck-Berechnung auf Grafikprozessoreinheit(en)), ein Parallelrechner (zum gleichzeitigen Ausführen, unter anderem auf mehreren Hauptprozessoren und/oder Grafikprozessoren, von Rechenoperationen) oder ein DSP realisiert sein.

Es ist dem Fachmann zu dem klar, dass auch dann wenn die hierin beschriebenen Details in Bezug auf ein Verfahren beschrieben werden, diese Details auch in einer geeigneten Vorrichtung, einem Computerprozessor oder einem mit einem Prozessor verbundenen Speicher realisiert sein können, wobei der Speicher mit einem oder mehreren Programmen versehen ist, die das Verfahren durchführen, wenn sie durch den Prozessor ausgeführt werden. Hierbei können Verfahren wie Swapping (zu Deutsch: Umlagerung) und Paging (zu Deutsch: Kachelverwaltung) verwendet werden.

Auch wenn einige der voranstehend beschriebenen Aspekte in Bezug auf die Vorrichtung beschrieben wurden, so können diese Aspekte auch auf das Verfahren zutreffen. Genauso können die voranstehend in Bezug auf das Verfahren beschriebenen Aspekte in entsprechender Weise auf die Vorrichtung zutreffen.

Heißt es vorliegend, dass eine Komponente mit einer anderen Komponente "verbunden ist", damit "in Verbindung steht" oder "darauf zugreift", kann dies heißen, dass sie damit unmittelbar verbunden ist oder auf diese unmittelbar zugreift; hierbei ist aber anzumerken, dass eine weitere Komponente dazwischenliegen kann. Heißt es andererseits, dass eine Komponente mit einer anderen Komponente "unmittelbar verbunden" ist oder "unmittelbar darauf zugreift", ist darunter zu verstehen, dass dazwischen keine weiteren Komponenten vorhanden sind.

### Kurzbeschreibung der Figuren

Die Erfindung wird nachfolgend mit Hilfe von Zeichnungen erläutert. Es zeigen:
- Figur 1: eine schematische Darstellung einer medizinischen Vorrichtung, nämlich einer Infusionspumpe, als Beispiel für eine Vorrichtung für eine sicherheitsrelevante Anwendung mit grafischer Anzeige und Touchscreen-Benutzeroberfläche;
- Figur 2: eine schematische Darstellung einer grundlegenden Gestaltung eines Editors;
- Figur 3: eine schematische Darstellung eines Editors mit verschiebbarem Dezimalpunkt;
- Figur 4: eine schematische Darstellung eines Editors mit verschobenem Dezimalpunkt (nur Vorkommastellen);
- Figur 5: eine schematische Darstellung eines Zeit-Editors;
- Figur 6: eine schematische Darstellung eines Editors mit ausgewähltem Digit und numerischer Tastatur;
- Figur 7: eine schematische Darstellung eines Editors mit spezifiziertem Digit;
- Figur 8: eine schematische Darstellung eines Editors mit Begrenzungsbalken; und
- Figur 9: eine schematische Darstellung eines Editors mit Soft-Limit-Warnung.

Die Figuren sind lediglich schematischer Natur und dienen ausschließlich dem Verständnis der Erfindung. Die gleichen Elemente sind mit denselben Bezugszeichen versehen. Die Merkmale der einzelnen Ausführungsformen können untereinander ausgetauscht werden.

Darüber hinaus können hier räumlich relative Begriffe, wie etwa "darunter befindlich", "unter", "untere(r)"/"unteres", "darüber befindlich", "obere(r)"/"oberes", "links", "linke(r)/linkes", "rechts", "rechte(r)/rechtes" und dergleichen, zur einfachen Beschreibung der Beziehung eines Elements oder einer Struktur zu einem oder mehreren anderen Elementen oder Strukturen verwendet werden, die in den Figuren dargestellt sind. Die räumlich relativen Begriffe sollen zusätzlich zu der in den Figuren dargestellten Orientierung andere Orientierungen des in Gebrauch oder in Betrieb befindlichen Bauelements umfassen. Das Bauelement kann anders ausgerichtet werden (um 90 Grad gedreht oder in einer anderen Orientierung), und die räumlich relativen Deskriptoren, die hier verwendet werden, können ebenso entsprechend interpretiert werden.

### Figurenbeschreibung

Das Verfahren und die Vorrichtung werden nun beispielhaft anhand von Ausführungsformen beschrieben.

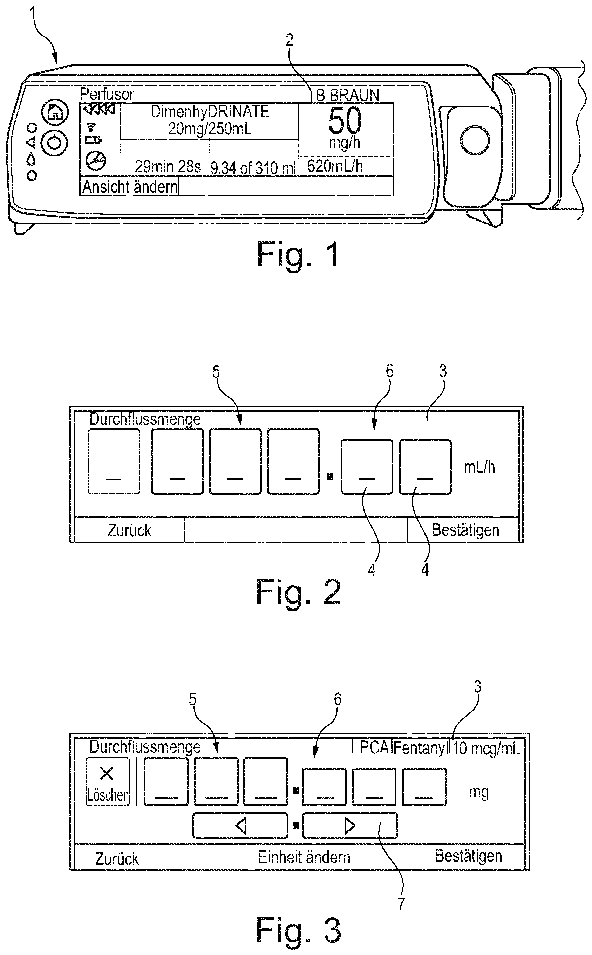

Das Konzept zielt auf sicherheitskritische Anwendungen, insbesondere in Bezug auf medizinische Vorrichtungen wie die Infusionspumpe 1 mit Touchscreen-Benutzeroberfläche 2 in Figur 1, oben auch als die Benutzerschnittstelle 2 beschrieben oder als Teil davon, ab, das die Eingabe von numerischen Daten durch einen Benutzer, auch Anwender genannt, erfordert.

Der Editor 3 als Interaktionswerkzeug bzw. Eingabewerkzeug für den Mensch-Maschine-Vorgang als Teil der oben beschriebenen Benutzerschnittstelle 2 ist im Hinblick auf die Sicherheit konzipiert. Ein Interaktionsprinzip beinhaltet einen Mechanismus, bei dem der Benutzer gezwungen wird, seine Aufmerksamkeit auf den Eingabeprozess zum Beispiel bei Eingabe von Werten für eine Infusionspumpe 1, wie in Figur 1 gezeigt, zu maximieren, um dadurch Fehler zu vermeiden.

Die Grundidee ist die Aufteilung einer Zahl in Ziffernfelder 4, hierin als Digits bezeichnet, die vom Benutzer individuell ausgewählt und mit einem numerischen Wert belegt werden müssen. Dieser Ansatz zwingt den Benutzer, während des Editierens über die Zahl nachzudenken, während er sich visuell auf die relevanten Bildschirmelemente des Editors 3 konzentriert.

Insbesondere sind hierbei die Ziffernfelder 4 interaktiv ausgestaltet.

Der Editor 3 verwendet eine Reihe von Ziffernfeldern 4, die es ermöglichen, während der Programmierung individuell Zahlenwerte anzugeben (siehe Figur 2).

Die Vorkommastellen 5 und die Nachkommastellen 6 sind durch einen Dezimalpunkt getrennt, der es dem Benutzer ermöglicht, die Vorkommastellen 5 und die Nachkommastellen 6 klar zu unterscheiden. Die Nachkommastellen sind zur weiteren visuellen Unterscheidung kleiner dargestellt. Der Abstand zwischen den Ziffernfeldern 4 kann vergrößert sein, um zwischen Hunderterstellen und Tausenderstellen zu unterscheiden.

Zur Bearbeitung freigegebene Ziffernfelder 4 können farbig, zum Beispiel weiß hinterlegt, dargestellt sein und durch Berühren der Touchscreen-Benutzeroberfläche 2 an diesen Stellen können Werte eingegeben werden. Ziffernfelder 4, die aus verschiedenen Gründen nicht bearbeitet werden können (z.B. aufgrund von Grenzwertbeschränkungen, die sich aus der Gerätephysik oder der Anwendung ergeben), werden zum Beispiel anders farbig hinterlegt, zum Beispiel ausgegraut. Der Benutzer kann daher nur Werte eingeben, die bestimmten Grenzwerten entsprechen oder in zulässige Bereiche fallen.

Der Editor 3, wie in Figur 2 dargestellt, kann beispielweise maximal sechs Ziffernfelder 4 anzeigen. Der Benutzer kann jedoch möglicherweise nicht alle sechs Ziffernfelder 4 bearbeiten, bei denen eine Anwendungslogik die Angabe von eingeschränkten oder unlogischen Werten verhindert. Wenn Nachkommastellen 6 verwendet werden, kann der Editor 3 den Benutzer dazu zwingen, maximal vier aufeinanderfolgende Ziffernfelder 4 zu bearbeiten, so dass der Benutzer keinen unlogischen Wert wie "1000.01" eingeben kann. Vielmehr können Werte wie "10.25" oder "110.5" von der Anwendungslogik als konform angesehen werden. Wo nur Vorkommastellen 5 zur Angabe eines Wertes erforderlich sind, kann der Editor 3 auch ohne den Dezimalpunkt dargestellt werden.

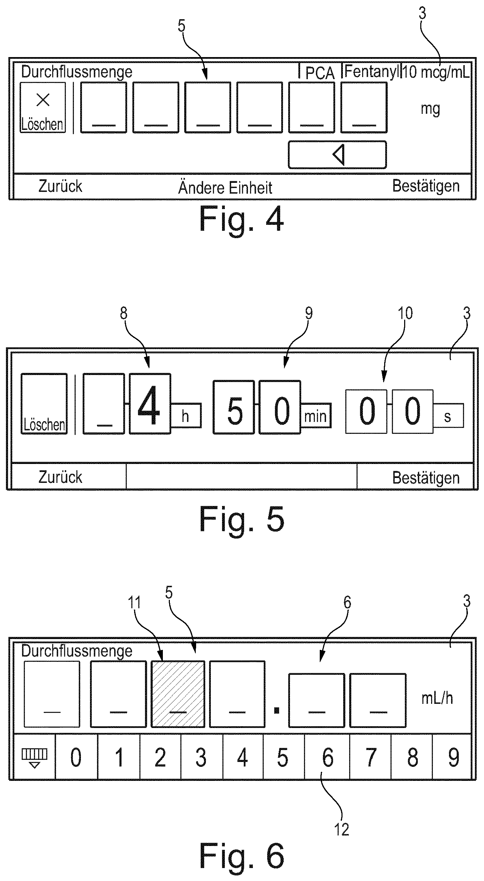

Wie in den Figuren 3 und 4 schematisch dargestellt, kann der Editor 3 einen beweglichen Dezimalpunkt aufweisen. In einem Anwendungsszenario bleibt der Dezimalpunkt an einer festen Position und kann nicht verschoben werden. Einige Szenarien erfordern jedoch die Flexibilität, mehr oder weniger Ziffernfelder 4 entweder rechts oder links vom Dezimalpunkt anzugeben (siehe Figur 3). Für diese Fälle erlaubt der Editor 3 dem Benutzer, den Dezimalpunkt an eine andere Position zu verschieben über die Pfeile zum Bewegen des Dezimalpunkts 7. Der Dezimalpunkt kann niemals ohne bewusste Handlung des Benutzers verschoben werden.

Das Verschieben des Dezimalpunkts ändert nicht die allgemeine Funktion des Editors 3. Alle zusätzlichen Nachkommafelder 6 und Ziffernfelder 4 sind kleiner, um die Unterscheidung von den Vorkommastellen 5 zu gewährleisten. Die Schaltflächen 7 zum Verschieben des Dezimalpunktes bewegen sich ebenfalls an der Stelle des Dezimalpunktes, um die Klarheit der Dezimalpunktpositionierung zu erhöhen. Sobald ein Wert eingegeben wurde, verschwinden die Pfeile 7 zum Verschieben des Dezimalpunktes.

Wie in Figur 5 dargestellt, kann ebenfalls eine Bearbeitung von Zeitinformationen vorgesehen sein. Zeit-Editoren 3 können dem gleichen Schema folgen und haben eine Aufteilung in Stunden 8 als Obereinheit, Minuten 9 als Mitteneinheit und Sekunden 10 als Untereinheit, unabhängig davon, ob sie editierbare Ziffernfelder 4 enthalten oder nicht. Die gleichzeitige Anzeige von Stunden 8, Minuten 9 und Sekunden 10 erfolgt mit dem Ziel, den Benutzer davor zu bewahren, Stunden 8 mit Minuten 9 und Minuten 9 mit Sekunden 10 zu verwechseln. Wenn die Eingabe von Stunden 8, Minuten 9 oder Sekunden 10 von der Anwendungslogik nicht erlaubt ist, werden die entsprechenden Ziffernfelder 4 andersfarbig hinterlegt, zum Beispiel ausgegraut (siehe Figur 5).

In einer möglichen Variante kann der Benutzer wahlweise entweder Stunden 8 in Kombination mit Minuten 9 oder Minuten 9 in Kombination mit Sekunden 10 eingeben. Wenn die Bearbeitung zunächst mit der Eingabe der Ziffernfelder 4 für die Stunden 8 beginnt, werden die Ziffernfelder 4 für die Sekunden 10 ausgegraut, also stehen nicht mehr zur Bearbeitung zur Verfügung. Dadurch wird die Verwirrung zwischen Stunden 8, Minuten 9 und Sekunden 10 verringert.

Das Auswählen eines Ziffernfeldes 4 bzw. das allgemeine Nutzungskonzept der Implementierung des numerischen Editors 3 erfordert, dass der Benutzer zunächst ein bestimmtes Ziffernfeld 4, das eine numerischen Werteingabe erfordert, berührt.

Bei Auswahl eines der Ziffernfelder 4 als ausgewähltes Ziffernfeld 11 erscheint unten im Editor 3 ein Ziffernblock in Form einer (virtuellen) ein-/ausklappbaren Tastatur 12, die zuvor ausgeblendet war (siehe Figur 6). Es zeigt alle Werte an, die an der ausgewählten Position, ausgewähltes Ziffernfeld 11, eingegeben werden können. Werte, die an der ausgewählten Position 11 aufgrund von Grenzwerten oder anderen Einschränkungen nicht verwendet werden dürfen, werden auf dem Ziffernblock ausgegraut, stehen somit also nicht zur Verfügung.

Wenn der Benutzer einen Wert für das ausgewählte Ziffernfeld 11 eingegeben hat, erscheint die Zahl im Ziffernfeld 11 und die virtuelle Tastatur 12 verschwindet. Der Editor 3 füllt alle Vorkommafelder 5 rechts von der angegebenen Vorkommastelle 5 mit Nullen 13, um die manuelle Eingabe von Nullen 13 zu vermeiden. Wenn der Benutzer keine Nullen 13 an einem bestimmten Ziffernfeld 4 wünscht, kann er dieses Ziffernfeld 4 auswählen, um dessen Wert zu ändern. In allen Fällen füllt der Editor 3 nur Ziffernfelder 4 mit Nullen 13, bei denen keine harten Grenzen verletzt werden.

Der Vorgang zur Auswahl eines Ziffernfeldes 4 wird so lange wiederholt, bis der Benutzer den vollständigen Wert für den gewünschten zur Anwendung vorgesehenen Parameter angegeben hat.

Nachdem der Benutzer einen Wert festgelegt hat, muss dieser durch Berühren der "Bestätigen"-Taste bestätigt werden, bevor die weitere Verarbeitung erfolgt. Die Wahl, den angegebenen Wert nicht zu verwenden, kann durch Berühren der Schaltfläche "Abbrechen" getroffen werden. In diesem Fall ignoriert die Vorrichtung, zum Beispiel die Infusionspumpe 1 aus Figur 1, die vom Editor 3 vorgenommenen Änderungen und kehrt zum vorherigen Wert (falls verfügbar) für diesen Parameter zurück.

Um einen Wert im Editor 3 zu löschen und neu zu beginnen, kann der Benutzer die Schaltfläche "Löschen" 14 berühren, die alle Werte der Ziffernfelder 4 löscht.

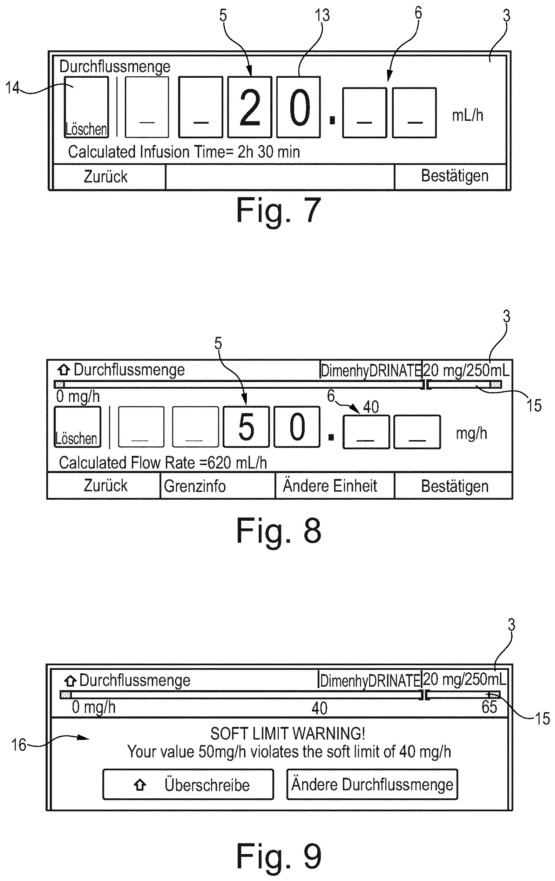

Der Editor 3 enthält das Konzept der Grenzen, die durch den so genannten Begrenzungsbalken 15 visuell angezeigt werden. Der Begrenzungsbalken 15 wird über dem numerischen Wert auf der in Figur 8 dargestellten Darstellung platziert.

Der Begrenzungsbalken 15 zeigt an seinen Enden die nicht zulässigen Wertebereiche an, die als harte Grenzen, auch in Englisch hard limits genannt, bezeichnet werden. Im mittleren Bereich des Balkens befindet sich der Standardwertebereich (zum Beispiel farbig gekennzeichnet, grauer Bereich) und der weiche Grenzbereich (zum Beispiel farbig gekennzeichnet, gelber Bereich). Der weiche Grenzbereich kennzeichnet Werte, auch in Englisch soft limits genannt, die zwar zulässig sind, aber aus Sicherheitsgründen besonders zu beachten/zu bestätigen sind.

Anwendungsspezifische Grenzwerte einer Infusionspumpe 1 können durch die Arzneimittelbibliothek bereitgestellt werden, die eine Sammlung von arzneimittel- und krankenhausbezogenen Sicherheitsinformationen darstellt, die in einem Speicher der Infusionspumpe abgelegt sind.

Die Überschreitung harter Grenzwerte wird durch den Editor 3 nicht zugelassen, da bei Auswahl bestimmter Ziffernfelder 4 oder darin eingegebener Werte, die harte Grenzwerte verletzen oder einer ungültigen Zahleneingabe entsprechen, der Fortschritt im Verfahrensablauf verzögert bzw. verhindert wird.

Wenn der Benutzer einen Wert angibt, der innerhalb des weichen Grenzwertbereichs für den Parameter liegt, reagiert die Infusionspumpe 1, indem der Bereich des Begrenzungsbalkens 15 andersfarbig, zum Beispiel gelb, markiert wird, der außerhalb der weichen Grenzen liegt (siehe Figur 8). Ein dynamischer Cursor auf dem Balken zeigt an, wo im Bereich der möglichen Werte der vom Benutzer eingegebene Wert liegt. Dies dient zur Information des Benutzers, bevor der Wert bestätigt wird. Für Infusionspumpen 1 können in der Medikamentenbibliothek des Geräts weiche Grenzwerte für ein bestimmtes Medikament festgelegt sein.

Wenn der Benutzer einen Wert bestätigt, der außerhalb einer weichen Grenze liegt, zeigt die Benutzeroberfläche 2 eine Soft-Limit-Warnmeldung 16 an, um zusätzliche Sicherheit zu gewährleisten. Die Soft-Limit-Warnmeldung 16 fordert den Benutzer auf, entweder die Soft-Limit-Warnung 16 zu überschreiben (d.h. den Wert zu akzeptieren) oder zur Bearbeitung des Wertes zurückzukehren, um ihn zu ändern (siehe Figur 9).

### Bezugszeichenliste

- 1: Infusionspumpe
- 2: Benutzeroberfläche
- 3: Editor
- 4: Ziffernfelder
- 5: Vorkommastellen
- 6: Nachkommastellen
- 7: Pfeile zum Bewegen des Dezimalpunktes
- 8: Stunden
- 9: Minuten
- 10: Sekunden
- 11: ausgewähltes Ziffernfeld
- 12: aufklappbare Tastatur
- 13: Nullen
- 14: Löschen
- 15: Begrenzungsbalken
- 16: Soft-Limit-Warnung

## Patentansprüche

1. Vorrichtung (1) für eine sicherheitsrelevante, vorzugsweise medizinische Anwendung mit einer Benutzerschnittstelle (2, 3), die vorgesehen ist, eine vorbestimmte Anzahl an Digits (4) zur Eingabe eines numerischen Parameters anzuzeigen, wobei die Benutzerschnittstelle (2, 3) ausgebildet ist, die vorbestimmte Anzahl an Digits (4) abhängig von Grenzwerten des numerischen Parameters oder mindestens einer anderen sicherheitsrelevanten Einschränkung auf eine Auswahl für eine Benutzereingabe zur Verfügung stehender Digits (4) zu beschränken, und einen numerischen Wert durch den Benutzer an einem der für eine Benutzereingabe zur Verfügung stehenden Digits (4) einzugeben.

2. Vorrichtung (1) nach Anspruch 1, wobei die Vorrichtung (1) eine medizinische Vorrichtung (1), vorzugsweise eine Infusionspumpe oder eine Dialysemaschine, ist.

3. Vorrichtung (1) nach Anspruch 1 oder 2, wobei die Benutzerschnittstelle (2, 3) ein Touchpad zur manuellen Ein-/Ausgabe und/oder eine Maus zur Eingabe aufweist.

4. Vorrichtung (1) nach einem der vorhergehenden Ansprüche, wobei die Benutzerschnittstelle (2, 3) ein Touchscreen zur manuellen Ein-/Ausgabe aufweist.

5. Vorrichtung (1) nach einem der vorhergehenden Ansprüche, wobei die für eine Benutzereingabe zur Verfügung stehenden Digits (4) separat und/oder nacheinander mit numerischen Werten belegbar sind.

6. Vorrichtung (1) nach einem der vorhergehenden Ansprüche, wobei jeder einzelne Digit (4) aus den zur Verfügung stehenden Digits (4) separat auswählbar ist und die Benutzerschnittstelle dafür vorgesehen und ausgebildet ist, mit oder durch Auswahl des jeweiligen Digits (4) eine virtuelle für alle auswählbaren Digits (4) allgemein gültige oder für das aktuell ausgewählte Digit (4) individuell ausgebildete oder belegte Tastatur (12) bereitzustellen vorzugsweise sichtbar zu machen weiter vorzugsweise aufzuklappen, über die dem aktuell ausgewählten Digit (4) ein numerischer Wert zuordenbar ist.

7. Vorrichtung (1) nach einem der vorhergehenden Ansprüche, wobei die Benutzerschnittstelle (2, 3) ausgebildet ist, die Auswahl der für die Benutzereingabe zur Verfügung stehenden Digits (4) auf eine gleiche Anzahl an Digits (4) für Vorkommastellen und Nachkommastellen oder eine gleiche Anzahl an Digits (4) für eine Einheit und eine Untereinheit, vorzugsweise Stunden und Minuten bzw. Minuten und Sekunde, zu verteilen.

8. Vorrichtung (1) nach einem der vorhergehenden Ansprüche, wobei die Benutzerschnittstelle (2, 3) ausgebildet ist, einen auf der Benutzerschnittstelle (2, 3) zur Anzeige von Vorkommastellen und Nachkommastellen vorgesehenen Dezimalpunkt zwischen den für eine Benutzereingabe zur Verfügung stehenden Digits (4) durch den Benutzer zu verschieben.

9. Vorrichtung (1) nach einem der vorhergehenden Ansprüche, wobei die für eine Benutzereingabe zur Verfügung stehenden Digits (4) Digits für Vorkommastellen und Digits für Nachkommastellen aufweist, wobei die Benutzerschnittstelle (2, 3) ausgebildet ist, alle Vorkommastellen rechts von der/den eingegebenen Vorkommastelle/Vorkommastellen mit Nullen zu füllen, um die manuelle Eingabe von Nullen zu vermeiden.

10. Vorrichtung (1) einem der vorhergehenden Ansprüche, wobei die Vorrichtung (1) einen Speicher aufweist, der vorgesehen ist, Daten umfassend mindestens einen harten numerischen Grenzwert und/oder einen weichen numerischen Grenzwert in Bezug auf die sicherheitsrelevante Anwendung zu speichern, wobei die Benutzerschnittstelle (2, 3) in Kommunikation mit dem Speicher steht, und ausgebildet ist, basierend auf den harten und/oder weichen numerischen Grenzwerten, einen Eingabefortschritt zu verzögern oder zu verhindern.

11. Vorrichtung (1) nach Anspruch 10, wobei die Benutzerschnittstelle (2, 3) ausgebildet ist, basierend auf einem Vergleich zwischen mindestens einem der numerischen Werte an mindestens einem der für eine Benutzereingabe zur Verfügung stehenden Digits (4) und dem mindestens einen harten numerischen Grenzwert:
eine weitere Eingabe zu beschränken oder eine vorherige Eingabe rückgängig zu machen.

12. Vorrichtung (1) nach Anspruch 10, wobei die Benutzerschnittstelle (2, 3) ausgebildet ist, eine Auswahl zur Verfügung stehender numerischer Werte an mindestens einem der für eine Benutzereingabe zur Verfügung stehenden Digits (4) basierend auf dem mindestens einen harten numerischen Grenzwert zu beschränken.

13. Vorrichtung (1) nach Anspruch 10, wobei die Benutzerschnittstelle (2, 3) ausgebildet ist, basierend auf einem Vergleich zwischen mindestens einem der numerischen Werte an mindestens einem der für eine Benutzereingabe zur Verfügung stehenden Digits (4) und dem mindestens einen weichen Grenzwert, mitzuteilen, dass eine weitere Eingabe erforderlich ist.

14. Verfahren zum Bedienen einer Vorrichtung (1) für eine sicherheitsrelevante Anwendung, vorzugsweise eine Vorrichtung (1) gemäß einem der vorhergehenden Ansprüche, das Verfahren umfassend:
Anzeigen, durch eine Benutzerschnittstelle (2, 3), der vorbestimmten Anzahl an Digits (4) zur Eingabe eines numerischen Parameters;
Beschränken, durch die Benutzerschnittstelle (2, 3), der vorbestimmten Anzahl an Digits (4) auf eine Auswahl für eine Benutzereingabe zur Verfügung stehender Digits (4) basierend auf Grenzwerten des numerischen Parameters oder mindestens einer anderen sicherheitsrelevanten Einschränkung; und
Eingeben eines numerischen Werts, durch einen Benutzer, an einem der zur Verfügung stehenden Digits (4).

## Claims

1. An apparatus (1) for a safety-relevant, preferably medical application, comprising a user interface (2, 3) intended to display a predetermined number of digits (4) for inputting a numerical parameter, wherein the user interface (2, 3) is configured to limit the predetermined number of digits (4) depending on limits of the numerical parameter or at least one other safety-relevant limitation to a selection of digits (4) available for a user input, and to input a numerical value by the user for one of the digits (4) available for a user input.

2. The apparatus (1) according to claim 1, wherein the apparatus (1) is a medical apparatus (1), preferably an infusion pump or a dialysis machine.

3. The apparatus (1) according to claim 1 or 2, wherein the user interface (2, 3) includes a touchpad for manual input/output and/or a mouse for input.

4. The apparatus (1) according to any of the preceding claims, wherein the user interface (2, 3) includes a touchscreen for manual input/output.

5. The apparatus (1) according to any of the preceding claims, wherein the digits (4) available for a user input can be filled separately and/or successively with numerical values.

6. The apparatus (1) according to any of the preceding claims, wherein each individual digit (4) can be separately selected from the available digits (4), and the user interface is intended and configured, with or by selecting the respective digit (4), to provide, preferably visualize, further preferably fold out a virtual keyboard (12) generally valid for all selectable digits (4) or individually designed or filled for the currently selected digit (4), via which keyboard a numerical value can be associated with the currently selected digit (4).

7. The apparatus (1) according to any of the preceding claims, wherein the user interface (2, 3) is configured to distribute the selection of the digits (4) available for user input to an equal number of digits (4) for pre-decimal point positions and post-decimal point positions, or to an equal number of digits (4) for a unit and a subunit, preferably hours and minutes and minutes and seconds, respectively.

8. The apparatus (1) according to any of the preceding claims, wherein the user interface (2, 3) is configured to shift a decimal point provided on the user interface (2, 3) for displaying pre- and post-decimal point positions between the digits (4) available for user input by the user.

9. The apparatus (1) according to any of the preceding claims, wherein the digits (4) available for user input have digits for pre-decimal point positions and digits for post-decimal point positions, the user interface (2, 3) being configured to zero-fill all pre-decimal point positions to the right of the pre-decimal point positions input so as to avoid manual input of zeros.

10. The apparatus (1) according to any of the preceding claims, wherein the apparatus (1) has a memory intended to store data comprising at least one hard numeric limit and/or one soft numeric limit with respect to the safety-relevant application, the user interface (2, 3) communicating with the memory and being configured to delay or prevent an input progress on the basis of the hard and/or soft numeric limits.

11. The apparatus (1) according to claim 10, wherein the user interface (2, 3) is configured, based on a comparison between at least one of the numerical values for at least one of the digits (4) available for a user input and the at least one hard numeric limit,
to restrict a further input or to reverse a previous input.

12. The apparatus (1) according to claim 10, wherein the user interface (2, 3) is configured to limit a selection of available numerical values for at least one of the digits (4) available for a user input based on the at least one hard numeric limit.

13. The apparatus (1) according to claim 10, wherein the user interface (2, 3) is configured, based on a comparison between at least one of the numerical values for at least one of the digits (4) available for a user input and the at least one soft limit, to notify that a further input is required.

14. A method for operating an apparatus (1) for a safety-relevant application, preferably an apparatus (1) according to any of the preceding claims, the method comprising:
Displaying, by a user interface (2, 3), the predetermined number of digits (4) for inputting a numerical parameter,
Limiting, by the user interface (2, 3), the predetermined number of digits (4) to a selection of digits (4) available for a user input based on limits of the numerical parameter or at least one other safety-relevant limitation; and
inputting a numerical value, by a user, for one of the available digits (4).

## Revendications

1. Dispositif (1) pour une application relative à la sécurité, de préférence médicale avec une interface utilisateur (2, 3) qui est prévue afin d'afficher un nombre prédéterminé d'éléments numériques (4) pour saisir un paramètre numérique, dans lequel l'interface utilisateur (2, 3) est conçue afin de limiter le nombre prédéterminé d'éléments numériques (4) en fonction de valeurs limites du paramètre numérique ou d'au moins une autre limitation relative à la sécurité à une sélection d'éléments numériques (4) se trouvant à disposition pour une saisie utilisateur, et de saisir une valeur numérique par l'utilisateur au niveau d'un des éléments numériques (4) se trouvant à disposition pour une saisie utilisateur.

2. Dispositif (1) selon la revendication 1, dans lequel le dispositif (1) est un dispositif médical (1), de préférence une pompe de perfusion ou une machine de dialyse.

3. Dispositif (1) selon la revendication 1 ou 2, dans lequel l'interface utilisateur (2, 3) présente un pavé tactile pour la saisie/la sortie manuelle et/ou une souris pour la saisie.

4. Dispositif (1) selon l'une quelconque des revendications précédentes, dans lequel l'interface utilisateur (2, 3) présente un écran tactile pour la saisie/la sortie manuelle.

5. Dispositif (1) selon l'une quelconque des revendications précédentes, dans lequel les éléments numériques (4) se trouvant à disposition d'une interface utilisateur peuvent être occupés séparément et/ou les uns après les autres par des valeurs numériques.

6. Dispositif (1) selon l'une quelconque des revendications précédentes, dans lequel chaque élément numérique (4) individuel des éléments numériques (4) se trouvant à disposition peut être sélectionné séparément, et l'interface utilisateur est prévue et conçue afin de fournir, de préférence de rendre visible, en outre de préférence ouvrir, un clavier (12) virtuel avec ou par la sélection de l'élément numérique (4) respectif généralement valable pour tous les éléments numériques (4) sélectionnables ou qui est occupé ou conçu individuellement pour l'élément numérique (4) sélectionné actuellement, clavier par le biais duquel une valeur numérique peut être associée à l'élément numérique (4) sélectionné actuellement.

7. Dispositif (1) selon l'une quelconque des revendications précédentes, dans lequel l'interface utilisateur (2, 3) est conçue afin de distribuer la sélection des éléments numériques (4) se trouvant à disposition pour la saisie utilisateur à un même nombre d'éléments numériques (4) pour des positions avant la virgule et des positions après la virgule ou un même nombre d'éléments numériques (4) pour une unité et une sous-unité, de préférence des heures et minutes ou des minutes et secondes.

8. Dispositif (1) selon l'une quelconque des revendications précédentes, dans lequel l'interface utilisateur (2, 3) est conçue afin de déplacer un point décimal prévu sur l'interface utilisateur (2, 3) pour afficher des positions avant la virgule et des positions après la virgule entre les éléments numériques (4) se trouvant à disposition pour une saisie utilisateur par l'utilisateur.

9. Dispositif (1) selon l'une quelconque des revendications précédentes, dans lequel les éléments numériques (4) se trouvant à disposition pour une saisie utilisateur présentent des éléments numériques pour des positions avant la virgule et des éléments numériques pour des positions après la virgule, dans lequel l'interface utilisateur (2, 3) est conçue afin de remplir par des zéros toutes les positions avant la virgule à droite de la/des position(s) avant la(les) virgule entrée(s) afin d'éviter la saisie manuelle de zéros.

10. Dispositif (1) selon l'une quelconque des revendications précédentes, dans lequel le dispositif (1) présente une mémoire qui est prévue afin d'enregistrer des données comprenant au moins une valeur limite numérique ferme et/ou une valeur limite numérique souple en ce qui concerne l'application relative à la sécurité, dans lequel l'interface utilisateur (2, 3) est en communication avec la mémoire et est conçue afin de retarder ou d'empêcher une progression de saisie sur la base des valeurs limites numériques fermes et/ou souples.

11. Dispositif (1) selon la revendication 10, dans lequel l'interface utilisateur (2, 3) est conçue afin de limiter, sur la base d'une comparaison entre au moins l'une des valeurs numériques au niveau d'au moins l'un des éléments numériques (4) se trouvant à disposition pour une saisie utilisateur et la au moins une valeur limite numérique ferme :
une autre saisie ou d'annuler une saisie précédente.

12. Dispositif (1) selon la revendication 10, dans lequel l'interface utilisateur (2, 3) est conçue afin de limiter une sélection de valeurs numériques se trouvant à disposition au niveau d'au moins l'un des éléments numériques (4) se trouvant à disposition pour une saisie utilisateur sur la base de la au moins une valeur limite numérique ferme.

13. Dispositif (1) selon la revendication 10, dans lequel l'interface utilisateur (2, 3) est conçue afin de communiquer qu'une autre saisie est nécessaire sur la base d'une comparaison entre au moins l'une des valeurs numériques au niveau d'au moins l'un des éléments numériques (4) se trouvant à disposition pour une saisie utilisateur et la au moins une valeur limite souple.

14. Procédé d'utilisation d'un dispositif (1) pour une application relative à la sécurité, de préférence un dispositif (1) selon l'une quelconque des revendications précédentes, le procédé comprenant :
l'affichage par une interface utilisateur (2, 3) du nombre prédéterminé d'éléments numériques (4) pour la saisie d'un paramètre numérique ;
la délimitation par l'interface utilisateur (2, 3) du nombre prédéterminé d'éléments numériques (4) à une sélection d'éléments numériques (4) se trouvant à disposition pour une saisie utilisateur sur la base de valeurs limites du paramètre numérique ou d'au moins une autre délimitation relative à la sécurité ; et
la saisie d'une valeur numérique, par un utilisateur, au niveau d'un des éléments numériques (4) se trouvant à disposition.
